# EUROPEAN PATENT SPECIFICATION

(11) **EP 4 527 290 B1**
(45) Date of publication and mention of the grant of the patent: **24.06.2026**
(21) Application number: 24199422.7
(22) Date of filing: 10.09.2024
(51) Int. Cl.: A61B 5/11, A61B 5/00

(54) **SYSTEM AND METHOD FOR PREDICTING LIKELIHOOD OF FALLING OR DEGREE OF ANESTHESIA RECOVERY**
SYSTEM UND VERFAHREN ZUR VORHERSAGE DER WAHRSCHEINLICHKEIT DES FALLENS ODER DES GRADES DER NARKOSEERHOLUNG
SYSTÈME ET PROCÉDÉ DE PRÉDICTION DE PROBABILITÉ DE CHUTE OU DE DEGRÉ DE RÉCUPÉRATION D'ANESTHÉSIE

(30) Priority: 22.09.2023 KR 20230127331
(43) Date of publication of application: 26.03.2025
(73) Proprietor: Samsung Life Public Welfare Foundation, Seoul 04348 (KR)
(72) Inventor: KIM, Do Kyoon, 06351 Seoul (KR)
(74) Representative: Plougmann Vingtoft a/s

(56) References cited:
- CN-A- 113 688 736
- US-A1- 2016 065 909
- US-A1- 2019 326 013
- US-A1- 2022 211 310

## Description

### CROSS-REFERENCE TO RELATED APPLICATION(S)

This application is based on and claims priority under 35 U.S.C. 119 to Korean Patent Application No. 10-2023-0127331, filed on September 22, 2023, in the Korean Intellectual Property Office.

### BACKGROUND OF THE INVENTION

### 1. Field of the invention

The present disclosure relates to a system and method for predicting the likelihood of falling or the degree of anesthesia recovery and, specifically, to a system and method for predicting the likelihood of falling or the degree of anesthesia recovery by using an image-based recognizer and artificial intelligence.

### 2. Description of the Prior Art

In recent years, hospitals, especially general hospitals, have been enlarged. The enlargement of hospitals allows for a variety of high-quality services for patients, but such hospitals result in increased patient numbers, which makes it harder to closely care patients who are at risk of falling from beds or in hallways or who are recovering from anesthesia. Especially, high-risk groups are highly susceptible to falling and challenges during anesthesia recovery, requiring close monitoring for patients, but there are limitations due to manpower issues.

At present, medical staff are directly deployed onsite to monitor patients, but real-time response to patient falling and accidents during anesthesia recovery is difficult in places where constant presence or management of medical staff is difficult, such as hallways, bathrooms, and patient rooms. Moreover, adding medical staff or extending working hours to enhance the ability to respond is costly and may quickly increase staff fatigue, resulting in lower efficiency.

In this regard, Korean Patent Publication No. 10-2013-0118512 discloses a patient condition monitoring system through face recognition and a patient condition monitoring service providing method using the same. The prior art document above discloses features of acquiring patient face capture images by including a watch camera, receiving the patient face capture images and vital sign information to detect whether a patient is in an emergency situation, and creating the resultant message to transmit the message to terminals of medical staff and caregivers for patients. However, according to the prior art document, the emergency situation is detected using vital signs, requiring additional equipment, which cannot be applied to all patients, thereby failing to solve fundamental problems.

There is therefore a need for a system and method for predicting the likelihood of falling or the degree of anesthesia recovery, wherein an image-based recognizer, applicable with a camera alone, can be installed to automatically detect patient's certain motions and falling motions or detect the degree of anesthesia recovery and to simultaneously detect multiple patients.

### Prior Art Document

### Patent Document

Korean Patent Publication No. 10 -2013-0118512

US2016/065909A1, US2019/326013A1, CN113688736A and US2022/211310A1 show further relevant prior art.

### SUMMARY OF THE INVENTION

An aspect of the present disclosure is to provide a system and method for predicting the likelihood of falling or the degree of anesthesia recovery, capable of automatically detecting patient's certain motions and falling motions by installing an image-based recognizer.

Another aspect of the present disclosure is to provide a system and method for predicting the likelihood of falling or the degree of anesthesia recovery, which are applicable to any place where imaging equipment is installed, by using an image alone to predict the likelihood of falling or the degree of anesthesia recovery.

Still another aspect of the present disclosure is to provide a system and method for predicting the likelihood of falling or the degree of anesthesia recovery, capable of detecting the likelihood of falling or the degree of anesthesia recovery for multiple patients simultaneously.

In accordance with an aspect of the present disclosure, there is provided a system for predicting the likelihood of falling or the degree of anesthesia recovery, the system including: at least one camera installed at a predetermined location in a hospital to capture an image; a motion detector configured to detect the motion of a patient in the image; a face recognizer configured to recognize the face of the patient in the image to determine the identity of the patient, and recognize the expression and gaze of the patient; and a monitor configured to predict the likelihood of falling or the degree of anesthesia recovery by using an action of the patient detected by the motion detector, the identity of the patient determined by the face recognizer, and the expression and gaze of the patient.

Preferably, the monitor may include an expression recognition module configured to recognize an expression correlating with a situation involving the likelihood of falling or with the degree of anesthesia recovery and the intensity of the expression.

Preferably, the monitor may include a gaze recognition module configured to distinguish the recognized gaze of the patient as at least one of saccades, vergence movements, smooth pursuit movements, and vestibulo-ocular movements, according to the eye movement.

Preferably, the gaze recognition module may calculate the difference between the distinguished gaze of the patient and a normal gaze to detect an abnormal gaze movement.

Preferably, the monitor may include an action recognition module configured to detect the posture of the patient by detecting a patient area from the image captured by the camera and extracting the skeleton of the patient within the area.

Preferably, the action recognition module may recognize the meaning of each action stage through a detected change in posture of the patient.

Preferably, the action recognition module may calculate the difference between the detected posture of the patient and a normal posture according to the meaning of each action stage to detect an abnormal action.

Preferably, the monitor may predict the likelihood of falling or the degree of anesthesia recovery by using an artificial neural network trained with features of gaze directions and skeletal movements of the patient, extracted from images indicating the likelihood of falling or images indicating the occurrence of falling.

Preferably, the monitor may classify whether or not the determined identity of the patient belongs to either a high-risk group for falling or a high-risk group for anesthesia recovery, and makes a prediction with increased sensitivity if the patient belongs to the high-risk group.

In accordance with another aspect of the present disclosure, there is provided a method for predicting the likelihood of falling or the degree of anesthesia recovery, the method including: capturing an image by at least one camera installed at a predetermined location in a hospital; detecting the motion of a patient in the image; recognizing the face of the patient in the image to determine the identity of the patient, and recognizing the expression and gaze of the patient; and predicting the likelihood of falling or the degree of anesthesia recovery by using the action of the patient detected in the detecting of the motion of the patient, the identity of the patient determined in the recognizing of the expression and gaze of the patient, and the expression and gaze of the patient.

The present disclosure has the advantage of enabling a real-time and automatic detection of the likelihood of falling or the degree of anesthesia recovery even in environments where medical staff are absent or insufficient.

Furthermore, the present disclosure has the advantage of enabling a simultaneous detection of the likelihood of falling or the degree of anesthesia recovery of all patients captured by a camera.

### BRIEF DESCRIPTION OF THE DRAWINGS

The above and other aspects, features and advantages of the present disclosure will be more apparent from the following detailed description taken in conjunction with the accompanying drawings, in which:
FIG. 1 is a block diagram of a system for predicting the likelihood of falling or the degree of anesthesia recovery according to an embodiment of the present disclosure.
FIG. 2 is a block diagram of a monitor according to an embodiment of the present disclosure.
FIG. 3 is a specific flow chart illustrating predicting, by the monitor, the likelihood of falling or the degree of anesthesia recovery according to an embodiment of the present disclosure.
FIG. 4 illustrates that the monitor predicts the likelihood of falling by comprehensively considering the gaze direction and the skeletal movement according to an embodiment of the present disclosure.
FIG. 5 is a block diagram of an input-output structure of an artificial neural network used in the monitor according to an embodiment of the present disclosure.
FIG. 6 is a flow chart illustrating a method for predicting the likelihood of falling or the degree of anesthesia recovery according to an embodiment of the present disclosure.

### DETAILED DESCRIPTION OF THE EXEMPLARY EMBODIMENTS

Hereinafter, the present disclosure will be described in detail with reference to accompanying drawings. However, the present disclosure is not restricted or limited by exemplary embodiments. Like reference numerals presented in each drawing refer to like elements that perform substantially the same functions.

Objects and effects of the present disclosure may be naturally appreciated or more clear by the following description and the objects and effects of the present disclosure are not limited only by the following disclosure. Furthermore, in the following description, a detailed explanation of known techniques associated with the present disclosure may be omitted to avoid unnecessarily obscuring the subject matter of the present disclosure.

The terminology used herein is for the purpose of describing particular embodiments only and is not intended to be limiting of the disclosure. A singular expression includes a plural expression, unless otherwise specified. It is to be understood that the terms such as "comprise" or "has" are used herein to designate a presence of characteristic, number, step, operation, element, component, or a combination thereof, and not to preclude a presence or a possibility of adding one or more of other characteristics, numbers, steps, operations, elements, components or a combination thereof.

The terms such as "first" and "second" may be used to describe a variety of elements, but the elements should not be limited by such terms. The terms are used only for the purpose of distinguishing one element from another. For example, a first element may be referred to as a second element and vice versa without departing from the scope of the present disclosure, and similarly, the second element may be named the 1st element.

Unless defined otherwise, all the terminologies used herein including technical or scientific terminologies have the same meaning as those understood by a person having ordinary skill in the art to which the present disclosure belongs. Terminologies as defined in a generally used dictionary should be interpreted to have the same meaning as those of the terminologies in context in the related descriptions, and shall not be interpreted in an ideal or excessively formal meaning unless they are explicitly defined herein.

Elements are interpreted to include an ordinary error range even if not expressly stated. In describing a time relationship, for example, when the temporal order is described as "after", "subsequent", "next", and "before", a case which is not continuous may be included unless "just" or "direct" is used.

Hereinafter, the technical features of the present disclosure will be described in detail with reference to the accompanying drawings.

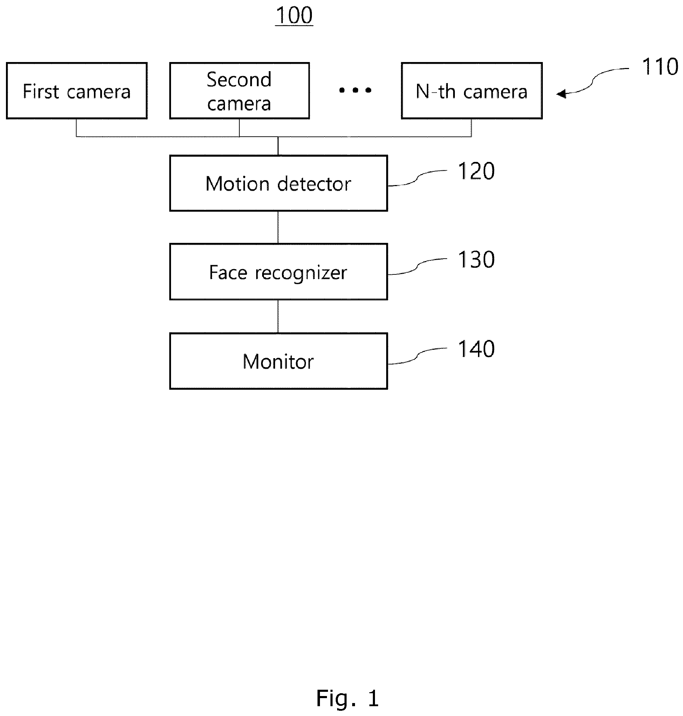

FIG. 1 is a block diagram of a system 100 for predicting the likelihood of falling or the degree of anesthesia recovery according to an embodiment of the present disclosure. Referring to FIG. 1, the system 100 for predicting the likelihood of falling or the degree of anesthesia recovery may include at least one camera 110, a motion detector 120, a face recognizer 130, and a monitor 140.

The system 100 for predicting the likelihood of falling or the degree of anesthesia recovery may detect the likelihood of falling or the degree of anesthesia recovery in real time and automatically even in environments where medical staff are absent or insufficient, thereby responding in real time to falling accidents of patients even in places where constant management is difficult, thus reducing the staff fatigue.

The system 100 for predicting the likelihood of falling or the degree of anesthesia recovery has the advantage of enabling the simultaneous detection of the likelihood of falling or the degree of anesthesia recovery of all patients captured by the camera.

The system 100 for predicting the likelihood of falling or the degree of anesthesia recovery may be installed in places where constant presence of medical staff is difficult, to automatically identify a patient, thereby predicting the likelihood of falling or the degree of anesthesia recovery in real time. The system 100 for predicting the likelihood of falling or the degree of anesthesia recovery may identify a patient, detect the likelihood of falling or the occurrence of falling, and transmit the detection information to the medical staff even in a space where the situation is unknown until medical staff actually enter, such as a bathroom in a patient room, with just a camera stalled therein. The system 100 for predicting the likelihood of falling or the degree of anesthesia recovery may optimally respond to patients belonging to a high-risk group for falling through specific patient linkage information (disease, medication status, etc.) in a specific patient room, from EMR data.

The system 100 for predicting the likelihood of falling or the degree of anesthesia recovery may simultaneously recognize the motion/face for each of multiple patients in the image, thus enabling the prediction of the likelihood of falling or the degree of anesthesia recovery for multiple patients in the image. In an embodiment, when multiple patients are monitored in a place where multiple patients and visitors co-exist, such as hospital hallways, the falling of a specific patient can be detected in the image through simultaneous motion/face recognition. Particularly, when a patient is determined as belonging to a high-risk group for falling (patients in a radiotherapy ward, patients administered medicine causing frequent urination, fracture patients, etc.) through face recognition and subsequent linkage of a patient degree, the system 100 for predicting the likelihood of falling or the degree of anesthesia recovery may lower the minimum threshold for falling motion recognition to take notice falling in advance even though the motion of the patient is minimal.

In another embodiment, when multiple beds are present and multiple patients are monitored in, for example, a patient room or an emergency center, the system 100 for predicting the likelihood of falling or the degree of anesthesia recovery may detect the occurrence of a falling event of a patient in a bed at a specific location in the image through automatic motion/face recognition. In still another embodiment, when multiple beds are present and multiple patients are monitored in, for example, a patient room or an emergency center, the system 100 for predicting the likelihood of falling or the degree of anesthesia recovery may detect the occurrence of a falling event of a patient in a bed at a specific location in the image even through automatic motion recognition alone. In still another embodiment, when multiple beds are present and the anesthesia conditions of multiple examinees are monitored in, for example, an endoscopy room of a medical examination center, the system 100 for predicting the likelihood of falling or the degree of anesthesia recovery may receive linkage information (anesthesia-associated examination history, examination items affecting anesthesia, etc.) of a specific examinee from EMR data through automatic face recognition (or a specific bed-examinee linkage information) for an examinee on each bed, to identify the degree of anesthesia recovery shown differently from each individual examinee, and automatically detects the motion of each specific examinee in the anesthesia recovery stage, and transmits the information, to medical staff, about whether or not the motion occurs within the normal recovery time range.

The system 100 for predicting the likelihood of falling or the degree of anesthesia recovery may include an image sensor, such as a camera. A radio frequency identification (RFID) or ultra-wideband (UWB) sensor may determine a person's identity, but when multiple persons move at the same time, the sensor cannot determine each individual's identity and cannot identify each individual's body motion, and moreover, each individual needs to carry an RFID or UWB sensing device. Radar may identify the body motion, but cannot determine human identities, individually. On the other hand, the system 100 for predicting the likelihood of falling or the degree of anesthesia recovery according to an embodiment of the present disclosure may determine the identities of several persons and the body motions of persons simultaneously, and each individual need not carry a sensing device.

The camera 110 may be installed at a predetermined location within a hospital to capture an image. One or more cameras 110 may be installed at a predetermined location within a hospital. The camera 110 may capture an image in real time and provide the captured image to the motion detector 120 or the face recognizer 130.

One or more cameras 110 may be disposed in places where there is a risk of falling of patients, such as corners, intersections, hallways, and patient rooms, or in anesthesia recovery rooms.

The motion detector 120 may detect the motion of a patient in the image. The motion detector 120 may recognize the motion of a patient, such as posture of a patient and a change in posture, through automatic motion recognition.

The motion detector 120 may detect the motion of a patient by recognizing the motion of a patient in real time. Specifically, the motion detector 120 may detect the motion of a patient by using artificial intelligence.

The face recognizer 130 may determine the identity of a patient by recognizing the face of the patient in the image. The face recognizer 130 may determine the identity of a patient by comparing the recognized face of the patient in an image and patient information on the database. The face recognizer 130 may determine the identity of a patient by recognizing the face of everyone as well as the face of the patient in an image. For example, the face recognizer 130 may determine the identities of patients, general visitors, medical staff, general employees, and the like.

The face recognizer 130 may determine the identity of a patient by recognizing the face of the patient in real time. Specifically, the face recognizer 130 may detect the motion of a patient by using artificial intelligence. The face recognizer 130 may offer high accuracy (99.9% or higher) that is resilient to temporal and environmental changes. The face recognizer 130 may provide a fast and easy identity verification function through highspeed/high-efficiency face recognition. The face recognizer 130 may provide a strong anti-spoofing function to distinguish whether the captured face is of a real person or a fake image. The motion detector 120 and the face recognizer 130 may utilize existing imaging devices, such as CCTV, installed in a hospital. The identity of a patient determined by the face recognizer 130 may be used to determine whether the identified patient belongs to a high-risk group for falling or anesthesia recovery.

The face recognizer 130 may recognize the expression and gaze of a patient. Particularly, the expression of the patient may refer to a look on the face that shows an emotion, such as grimacing, pain, or confusion, and the gaze of the patient may refer to the direction of the eyes of the patient.

The artificial intelligence for motion detection, face recognition, and monitoring mentioned above may be a Deep Neural Network (DNN) or Convolutional Neural Network (CNN)-based model. Convolutional neural network (CNN) is a type of deep learning model designed to process gridlike data, such as an image, and is inspired by the structure of the visual cortex in animals. The convolutional neural network may generally include convolutional layers, pooling layers, and fully connected layers. The convolutional layers and pooling layers may be repeated in a neural network, and input data may be converted to output through these layers. The convolution layers use a kernel (or mask) for feature extraction, where element-wise multiplication between each element of the kernel and the input values is performed and summed at each position to produce the output, which is referred to as the feature map. This procedure may be repeated with multiple kernels to form an arbitrary number of feature maps. In the convolutional neural network, the convolutional and pooling layers perform feature extraction, while the fully connected layers map the extracted features to the final output, such as classification operations.

The convolutional neural network may be trained to minimize output errors. Separately from forward propagation that extracts from the input layer to the output layer, backpropagation occurs in the neural network that calculates the error between the input training data and the output value of the neural network and updates the weights of the nodes in each layer to reduce the error. The training procedure in the convolutional neural network may be summarized as a procedure of finding a kernel that extracts the output value with the least error on the basis of the given training data. Kernel is the only parameter that is automatically trained during the training of the convolutional layers. However, in the convolutional neural network, the size of kernels, number of kernels, padding, and the like are hyper-parameters that need to be set before starting the training procedure, and therefore, different convolutional neural network models may be distinguished depending on the size of kernels, number of kernels, and numbers of convolutional layers and pooling layers.

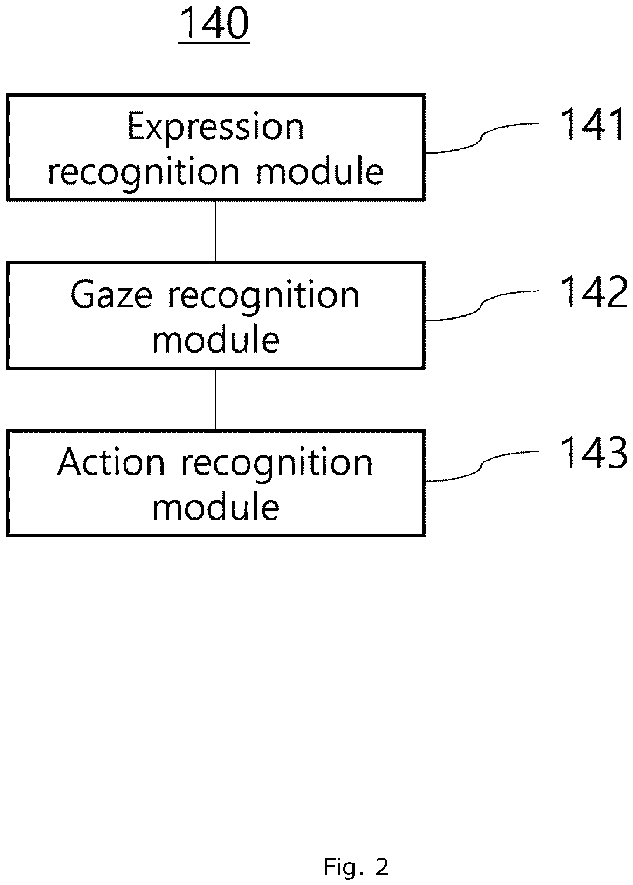

FIG. 2 is a block diagram of the monitor 140 according to an embodiment of the present disclosure. Referring to FIG. 2, the monitor 140 may include an expression recognition module 141, a gaze recognition module 142, and an action recognition module 143.

The monitor 140 may predict the likelihood of falling or the degree of anesthesia recovery by using the action of a patient detected by the motion detector 120, the identity of the patient determined by the face recognizer 130, and the expression and gaze of the patient. The monitor 140 may predict the likelihood of falling or the degree of anesthesia recovery by analyzing face recognition, such as identify determination, expression recognition, and gaze recognition, and motion recognition, such as posture, change in posture, a motion speed, and stop time. Unlike the conventional art, the monitor 140 may predict the likelihood of falling or the degree of anesthesia recovery according to the risk level at anytime and anywhere by using the captured image alone.

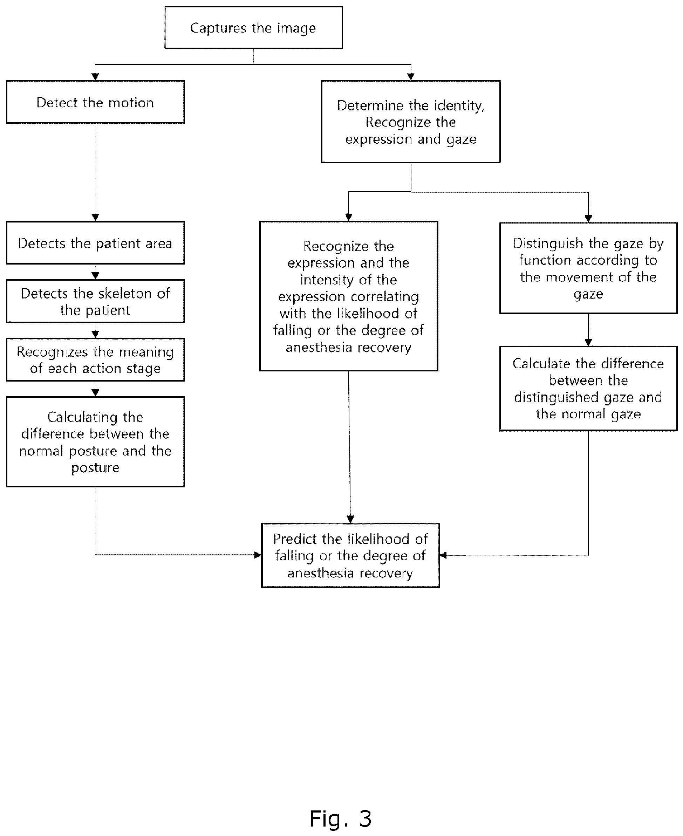

FIG. 3 is a specific flow chart illustrating predicting, by the monitor 140, the likelihood of falling or the degree of anesthesia recovery according to an embodiment of the present disclosure. Referring to FIG. 3, the monitor 140 may detect the motion of a patient in an image being captured in real time, determine the identity of the patient through face recognition, and recognize the expression and gaze. The monitor 140 detects a patient area, in the image, where a patient is present, detects the skeleton of the patient in the patient area to identify the posture, and then recognizes the meaning of each action stage through the change in posture. Then, the monitor 140 may detect the motion of the patient by calculating the difference between the normal posture and the posture of the patient, each of which has the meaning of each action stage.

The monitor 140 may recognize the expression correlating with the likelihood of falling or the degree of anesthesia recovery, and the intensity of the expression (the higher the intensity, the greater the correlation). The monitor 140 may also recognize the gaze of the patient by distinguishing the gaze by function according to the movement of the gaze and calculating the difference between the distinguished gaze and the normal gaze. Particularly, the determination of the identity of the patient may be used for only determination of a high-risk group.

The monitor 140 may finally integrate action recognition, expression recognition, and gaze recognition, thereby predicting the likelihood of falling or the degree of anesthesia recovery. The monitor 140 may predict the likelihood of falling in order to prevent falling and may also detect the occurrence of falling.

When the prediction of the likelihood of falling is performed in order to prevent falling, the monitor 140 may use an artificial neural network model previously trained with representative gaze directions, expressions, and human skeletal movements, which may cause falling. For example, a person looks at a location to move to or an object to hold, before taking a specific action, and then takes the action while continuing to look at the location or object. The monitor 140 may determine that a patient is at risk of falling if the representative gaze direction, expression, and action indicating a risk of falling, and may determine that a patient is at risk of falling by recognizing an issue with the synchronization of gaze and movement (a defect in vestibulo-ocular movements) in the event of a specific action.

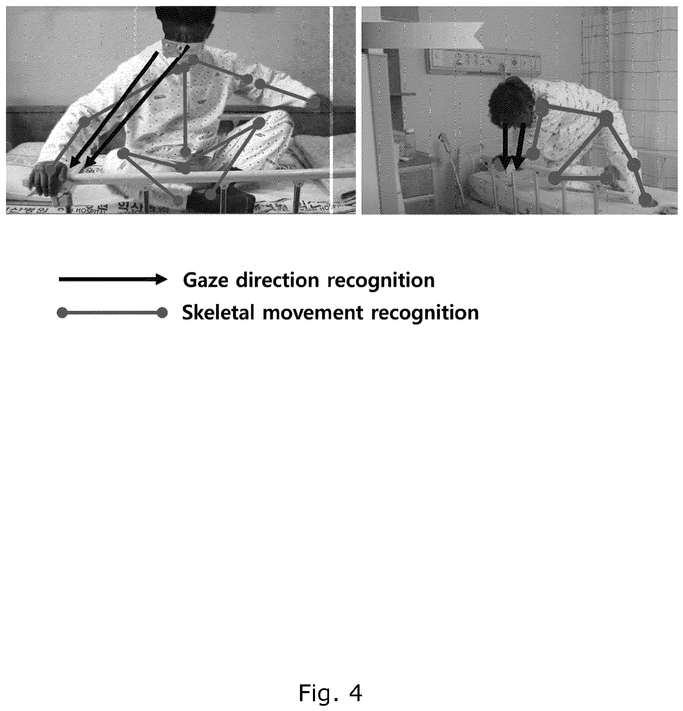

FIG. 4 illustrates that the monitor 150 predicts the likelihood of falling by comprehensively considering the gaze direction and the skeletal movement according to an embodiment of the present disclosure. Referring to FIG. 4, in an embodiment for predicting the likelihood of falling in order to prevent falling, an action of attempting to get out of the bed without lowering a side rail involves a motion of looking at the rail to hold the rail and getting up. Therefore, the monitor 140 may predict the risk of falling by training an artificial intelligence with this risk in advance or making this risk into a database. Additionally, the monitor 140 may detect the risk of falling in advance by recognizing that the gaze direction intended for holding the side rail is not consistently maintained toward the side rail according to the head or body movement due to a defect in vestibulo-ocular movements (the eye movement that occurs in the opposite direction of the head movement to keep the gaze directed and maintain equilibrium when the head moves).

In another embodiment, a motion of attempting to stretch out an arm from inside the bed to grab an object outside the bed involves an action of looking at the object to grab the object and stretching out the arm. Therefore, the monitor 140 may predict the risk of falling by training an artificial intelligence with this risk in advance or making this risk into a database.

In still another embodiment, the monitor 140 may predict the risk of falling by training an artificial intelligence with the following cases indicating the likelihood of falling in advance or making these cases into a database: when the bed rail has been lowered but a patient attempts to step onto the caregiver's bed (which may slide due to wheels) to get out of the bed while looking at the caregiver's bed; when a patient attempts to get out of the bed through the narrow space next to the side rail while looking at the space next to the side rail; when a patient attempts to get out of the bed alone and get on a wheelchair (which may slide due to wheels) while looking at the wheelchair; when a patient attempts to get up from a toilet without looking at or holding onto supports; when the gait of the patient is different from that of a normal person in ward hallways; and when a caregiver (who can be identified by face recognition) does not help the patient walk while looking at a smartphone in ward hallways.

To detect the occurrence of falling, the monitor 140 may use an artificial neural model that has been previously trained with representative gaze directions (floor or ceiling) and human skeletal movements (falling, tripping, slipping, etc.), which occur during or after falling. The monitor 140 may detect the occurrence of falling to allow medical staff to take prompt follow-up measures.

The expression recognition module 141 may recognize an expression correlated with the degree of anesthesia recovery or a situation involving the likelihood of falling, and the intensity of the expression. A specific expression of a patient is correlated with a specific situation. Examples of the specific expression may include grimacing, agonizing, fearful, confused, doubtful, eyelid closing, and expressionless, and dazed. The expression recognition module 141 may recognize the extent to which the current expression of a patient is correlated with the likelihood of falling or the degree of anesthesia recovery, by using the correlation between the degree of anesthesia recovery or a situation involving the likelihood of falling and an expression for each degree or situation, which has been stored in the database in advance. The expression recognition module 141 may output the extent to which the current expression of a patient is correlated with the likelihood of falling or the degree of anesthesia recovery, by using an artificial neural network trained with features of expressions of the patient according to the likelihood of falling or the degree of anesthesia recovery.

The gaze recognition module 142 may distinguish the recognized gaze of a patient as at least one of saccades, vergence movements, smooth pursuit movements, and vestibulo-ocular movements, according to the movement. Among the distinguished gaze movements, vergence movements, smooth pursuit movements, and vestibulo-ocular movements, excluding saccades are gaze functions greatly relevant to human conscious action. Therefore, the gaze recognition module 142 may recognize three types of gaze movements that are greatly relevant to human conscious action to determine an abnormal situation as a pre-symptom for a high risk of the likelihood of falling or anesthesia recovery. Particularly, a defect in vestibulo-ocular movements is directly correlated with falling, wherein the vestibulo-ocular movement refers to the gaze movement occurring in the opposite direction to the head movement to keep the gaze directed in a predetermined direction to maintain equilibrium when the head moves, and a defect in vestibulo-ocular movements is directly correlated with falling. The gaze recognition module 142 may distinguish the gaze movement from the current gaze of a patient by using an artificial neural network trained with features of any one or more of four types of gaze movements.

The gaze recognition module 142 may calculate a difference between the distinguished gaze of a patient and the normal gaze to detect an abnormal gaze movement. The gaze recognition module 142 may detect an abnormal gaze movement through the consistency of direction, intensity, and the like between the normal gaze movements stored in the database and the current gaze movement of a patient. The gaze recognition module 142 may detect an abnormal gaze movement by using an artificial neural network trained with features of normal or abnormal gaze movements.

The action recognition module 143 may detect a patient area from the image captured by the camera and extract the skeleton of the patient within the area to detect the posture of the patient. The action recognition module 143 may extract the skeleton of a patient in the image by using an artificial intelligence model. Specifically, the artificial intelligence model extracting the skeleton may extract the skeleton by extracting each joint of the human body and connecting the joints.

The action recognition module 143 may recognize the meaning of each action stage through the detected change in posture of the patient. The camera 110 may capture an image in real time and may detect the posture of a patient for each frame in the real-time captured image. Therefore, the action recognition module 143 may detect the change in posture of a patient over time by continuously comparing the postures of the patient detected for the respective frames, and may recognize the meaning of each action stage that the patient attempts, through continuous changes in posture. For example, the action recognition module 143 may recognize the meaning of each action stage through continuous changes in posture, such as an action of getting up the upper body from the bed or the ward chair, an action of holding the bed rail or chair armrest, an action of extending the legs to the side of the bed, an action of lowering the legs to the floor in the patient room while holding the bed floor or the bed rail, an action of raising the body while holding the chair armrest, an action of standing next to the bed or in front of the chair, an action of walking toward the exit or the bathroom, and an action of walking along ward hallways.

That is, the action recognition module 143 may recognize the meaning of each action stage by comparing the meaning of each action stage according to the change in posture stored in the database with the current change in posture of a patient. The action recognition module 143 may detect the meaning of each action stage by using an artificial neural network strained with features of the meaning of each action stage and the current change in posture of a patient.

The action recognition module 143 may calculate the difference between the detected posture of the patient and a normal posture, according to the meaning of each action stage, to detect an abnormal action. The action recognition module 143 may detect an abnormal action through the difference between a normal posture according to the meaning of each action stage stored in the database and the current posture of the patient. The action recognition module 143 may detect an abnormal action by using an artificial neural network trained with features of normal or abnormal gaze movements.

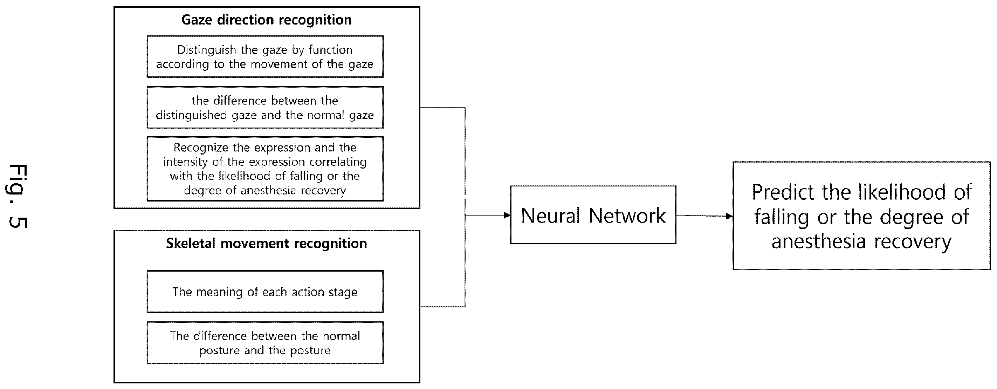

FIG. 5 is a block diagram of an input and output structure of an artificial neural network used in the monitor 140 according to an embodiment of the present disclosure. Referring to FIG. 5, the monitor 140 may predict the likelihood of falling by using an artificial neural network trained with features of gaze directions and skeletal movements of a patient, extracted from images indicating the likelihood of falling or images indicating the occurrence of falling. Particularly, the data used to train the artificial neural network may include features of gazes distinguished according to the movement, features of gaze directions including differences from normal gazes, or correlated expressions and expression intensity recognition, and features of skeletal movements including the meaning of each action stage and differences from normal postures.

The monitor 140 may classify the determined identity of the patient as belonging to a high-risk group for falling or anesthesia recovery, and may make a prediction with high sensitivity if the patient belongs to a high-risk group. The monitor 140 may utilize information about a high-risk group for falling or a high-risk group for anesthesia recovery that may undergo fatal injuries in the occurrence of falling or may be highly susceptible to falling, to predict the likelihood of falling or the degree of anesthesia recovery.

The monitor 140 can inform medical staff and caregivers of the stage of the likelihood of falling, rather than simply turning on/off for the likelihood of falling based on the results of the difference between an action and the meaning of the action. For example, the monitor 140 may inform the likelihood of falling for each action stage, or may inform the likelihood of falling by combining the results of the difference between an action stage and a normal action.

The monitor 140 may transmit, to medical staff, a falling risk stage, a falling occurrence notification, or an anesthesia recovery stage if the likelihood of falling, the occurrence of falling, or the degree of anesthesia recovery is detected. Additionally, the monitor 140 may optimally respond to a corresponding patient by also transmitting, to medical staff, specific patient linkage information (disease, medication status, etc.) in a specific patient room, from EMR data, when transmitting a falling risk stage, a falling occurrence notification, or an anesthesia recovery stage.

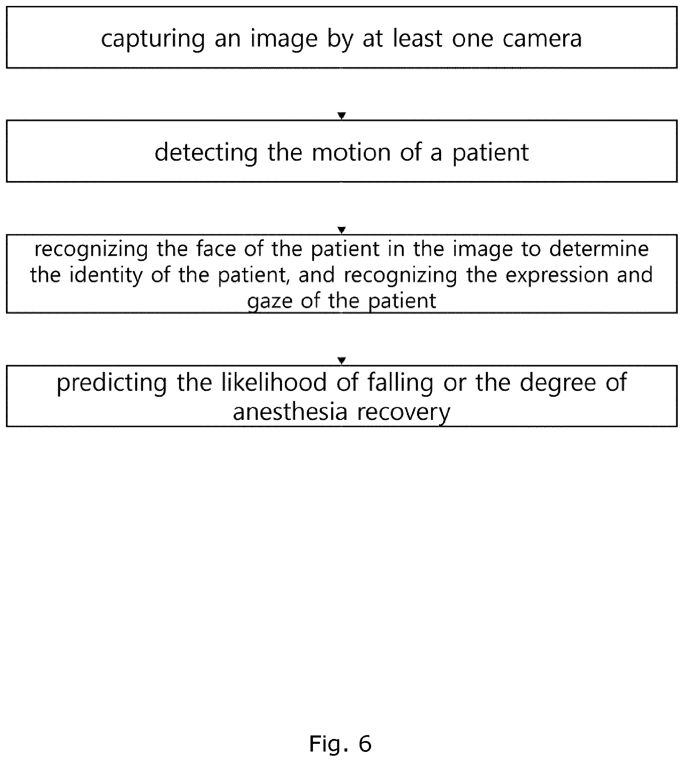

FIG. 6 is a flow chart illustrating a method for predicting the likelihood of falling or the degree of anesthesia recovery according to another embodiment of the present disclosure. Referring to FIG. 6, the method may include: capturing an image by at least one camera installed at a predetermined location in a hospital; detecting the motion of a patient in the image; recognizing the face of the patient in the image to determine the identity of the patient, and recognizing the expression and gaze of the patient; and predicting the likelihood of falling or the degree of anesthesia recovery by using the action of the patient detected in the detecting of the motion of the patient, the identity of the patient determined in the recognizing of the expression and gaze of the patient, and the expression and gaze of the patient.

The capturing of an image by at least one camera installed at a predetermined location in a hospital indicates the above-described operations performed by the at least one camera 110.

The recognizing of the face of the patient in the image to determine the identity of the patient and recognizing of the expression and gaze of the patient indicates the above-described operations performed by the face recognizer 130.

The predicting of the likelihood of falling or the degree of anesthesia recovery by using the action of the patient detected in the detecting of the motion of the patient, the identity of the patient determined in the recognizing of the expression and gaze of the patient, and the expression and gaze of the patient indicates the above-described operations performed by the monitor 104.

Although the present disclosure has been described in detail through the exemplary embodiments above, those skilled in the art to which the present disclosure pertains will understand that various modifications may be made to the above-described exemplary embodiments without departing from the scope of the present disclosure. Therefore, the scope of the present disclosure should not be limited to the described exemplary embodiments, and should be determined not only by the scope of the claims to be described later, but also by any changes or modifications within the scope of the claims.

## Claims

1. A system for predicting the likelihood of falling or the degree of anesthesia recovery, the system comprising:
at least one camera installed at a predetermined location in a hospital to capture an image;
a motion detector configured to detect the motion of a patient in the image;
a face recognizer configured to recognize the face of the patient in the image to determine the identity of the patient, and recognize the expression and gaze of the patient; and
a monitor configured to predict the likelihood of falling or the degree of anesthesia recovery by using an action of the patient detected by the motion detector, the identity of the patient determined by the face recognizer, and the expression and gaze of the patient.

2. The system of claim 1, wherein the monitor comprises an expression recognition module configured to recognize an expression correlating with a situation involving the likelihood of falling or with the degree of anesthesia recovery and the intensity of the expression.

3. The system of claim 1, wherein the monitor comprises a gaze recognition module configured to distinguish the recognized gaze of the patient as at least one of saccades, vergence movements, smooth pursuit movements, and vestibulo-ocular movements, according to the eye movement.

4. The system of claim 3, wherein the gaze recognition module calculates the difference between the distinguished gaze of the patient and a normal gaze to detect an abnormal gaze movement.

5. The system of claim 1, wherein the monitor comprises an action recognition module configured to detect the posture of the patient by detecting a patient area from the image captured by the camera and extracting the skeleton of the patient within the area.

6. The system of claim 5, wherein the action recognition module recognizes the meaning of each action stage through a detected change in posture of the patient.

7. The system of claim 6, wherein the action recognition module calculates the difference between the detected posture of the patient and a normal posture according to the meaning of each action stage to detect an abnormal action.

8. The system of claim 1, wherein the monitor predicts the likelihood of falling or the degree of anesthesia recovery by using an artificial neural network trained with features of gaze directions and skeletal movements of the patient, extracted from images indicating the likelihood of falling or images indicating the occurrence of falling.

9. The system of claim 1, wherein the monitor classifies whether or not the determined identity of the patient belongs to either a high-risk group for falling or a high-risk group for anesthesia recovery, and makes a prediction with increased sensitivity if the patient belongs to the high-risk group.

10. A method for predicting the likelihood of falling or the degree of anesthesia recovery, the method comprising:
capturing an image by at least one camera installed at a predetermined location in a hospital;
detecting the motion of a patient in the image;
recognizing the face of the patient in the image to determine the identity of the patient, and recognizing the expression and gaze of the patient; and
predicting the likelihood of falling or the degree of anesthesia recovery by using the action of the patient detected in the detecting of the motion of the patient, the identity of the patient determined in the recognizing of the expression and gaze of the patient, and the expression and gaze of the patient.

## Patentansprüche

1. System zur Vorhersage der Wahrscheinlichkeit des Fallens oder des Grades der Narkoseerholung, das System umfassend:
mindestens eine Kamera, die an einem vorbestimmten Ort in einem Krankenhaus installiert ist, um ein Bild aufzunehmen;
einen Bewegungsmelder, der dazu konfiguriert ist, die Bewegung eines Patienten im Bild zu erfassen;
einen Gesichtserkenner, der dazu konfiguriert ist, das Gesicht des Patienten im Bild zu erkennen, um die Identität des Patienten zu bestimmen, und den Ausdruck und den Blick des Patienten zu erkennen; und
einen Monitor, der dazu konfiguriert ist, durch Verwenden einer durch den Bewegungsmelder erfassten Handlung des Patienten, der durch den Gesichtserkenner bestimmten Identität des Patienten und des Ausdrucks und des Blick des Patienten die Wahrscheinlichkeit des Fallens oder den Grad der Narkoseerholung vorherzusagen.

2. System nach Anspruch 1, wobei der Monitor ein Ausdruckserkennungsmodul umfasst, das dazu konfiguriert ist, einen Ausdruck, der mit einer Situation, die die Wahrscheinlichkeit des Fallens involviert, oder mit dem Grad der Narkoseerholung korreliert, und die Intensität des Ausdrucks zu erkennen.

3. System nach Anspruch 1, wobei der Monitor ein Blickerkennungsmodul umfasst, das dazu konfiguriert ist, den erkannten Blick des Patienten entsprechend der Augenbewegung als mindestens eines von Sakkaden, Vergenzbewegungen, sanften Verfolgungsbewegungen und vestibulookulären Bewegungen zu unterscheiden.

4. System nach Anspruch 3, wobei das Blickerkennungsmodul die Differenz zwischen dem unterschiedenen Blick des Patienten und einem normalen Blick berechnet, um eine abnormale Blickbewegung zu erfassen.

5. System nach Anspruch 1, wobei der Monitor ein Handlungserkennungsmodul umfasst, das dazu konfiguriert ist, durch Erfassen eines Patientenbereichs aus dem durch die Kamera aufgenommenen Bild und Extrahieren des Skeletts des Patienten innerhalb des Bereichs die Körperhaltung des Patienten zu erfassen.

6. System nach Anspruch 5, wobei das Handlungserkennungsmodul durch eine erfasste Änderung der Körperhaltung des Patienten die Bedeutung jeder Handlungsphase erkennt.

7. System nach Anspruch 6, wobei das Handlungserkennungsmodul die Differenz zwischen der erfassten Körperhaltung des Patienten und einer normalen Körperhaltung entsprechend der Bedeutung jeder Handlungsphase berechnet, um eine abnormale Handlung zu erfassen.

8. System nach Anspruch 1, wobei der Monitor durch Verwenden eines künstlichen neuronalen Netzwerks, das mit Merkmalen der Blickrichtungen und Skelettbewegungen des Patienten trainiert wurde, die aus Bildern, die die Wahrscheinlichkeit des Fallens angeben, oder Bildern, die das Auftreten eines Fallens angeben, extrahiert wurden, die Wahrscheinlichkeit des Fallens oder den Grad der Narkoseerholung vorhersagt.

9. System nach Anspruch 1, wobei der Monitor klassifiziert, ob die bestimmte Identität des Patienten zu entweder einer Hochrisikogruppe zum Fallen oder einer Hochrisikogruppe zur Narkoseerholung gehört oder nicht, und eine Vorhersage mit erhöhter Sensitivität trifft, wenn der Patient zur Hochrisikogruppe gehört.

10. Verfahren zur Vorhersage der Wahrscheinlichkeit des Fallens oder des Grades der Narkoseerholung, das Verfahren umfassend:
Aufnehmen eines Bildes durch mindestens eine Kamera, die an einem vorbestimmten Ort in einem Krankenhaus installiert ist;
Erfassen der Bewegung eines Patienten im Bild;
Erkennen des Gesichts des Patienten im Bild, um die Identität des Patienten zu bestimmen, und Erkennen des Ausdrucks und des Blicks des Patienten; und
Vorhersagen der Wahrscheinlichkeit des Fallens oder des Grades der Narkoseerholung durch Verwendung der Handlung des Patienten, die beim Erfassen der Bewegung des Patienten erfasst wurde, der Identität des Patienten, die beim Erkennen des Ausdrucks und des Blicks des Patienten bestimmt wurde, und des Ausdrucks und des Blicks des Patienten.

## Revendications

1. Système de prédiction de probabilité de chute ou de degré de récupération d'anesthésie, le système comprenant :
au moins une caméra installée à un emplacement préétabli dans un hôpital pour capturer une image ;
un détecteur de mouvement configuré pour détecter le mouvement d'un patient dans l'image ;
une unité de reconnaissance faciale configurée pour reconnaître le visage du patient dans l'image afin de déterminer l'identité du patient, et reconnaître l'expression et le regard du patient ; et
un dispositif de surveillance configuré pour prédire la probabilité de chute ou le degré de récupération d'anesthésie en utilisant une action du patient détectée par le détecteur de mouvement, l'identité du patient déterminée par l'unité de reconnaissance faciale, et l'expression et le regard du patient.

2. Système selon la revendication 1, dans lequel le dispositif de surveillance comprend un module de reconnaissance d'expression configuré pour reconnaître une expression présentant une corrélation avec une situation impliquant la probabilité de chute ou avec le degré de récupération d'anesthésie, ainsi que l'intensité de l'expression.

3. Système selon la revendication 1, dans lequel le dispositif de surveillance comprend un module de reconnaissance du regard, configuré pour distinguer le regard reconnu du patient comme étant au moins l'un parmi : des saccades, des mouvements de vergence, des mouvements de poursuite régulière, et des mouvements oculo-vestibulaires, en fonction du mouvement oculaire.

4. Système selon la revendication 3, dans lequel le module de reconnaissance du regard calcule la différence entre le regard ainsi distingué du patient et un regard normal pour détecter un mouvement anormal du regard.

5. Système selon la revendication 1, dans lequel le dispositif de surveillance comprend un module de reconnaissance d'action configuré pour détecter la posture du patient en détectant une zone du patient à partir de l'image capturée par la caméra et en extrayant le squelette du patient à l'intérieur de la zone.

6. Système selon la revendication 5, dans lequel le module de reconnaissance d'action reconnaît la signification de chaque phase d'action par l'intermédiaire d'une variation détectée dans la posture du patient.

7. Système selon la revendication 6, dans lequel le module de reconnaissance d'action calcule la différence entre la posture détectée du patient et une posture normale en fonction de la signification de chaque phase d'action pour détecter une action anormale.

8. Système selon la revendication 1, dans lequel le dispositif de surveillance prédit la probabilité de chute ou le degré de récupération d'anesthésie en utilisant un réseau neuronal artificiel entraîné avec des caractéristiques de directions du regard et de mouvements squelettiques du patient, extraites d'images indiquant la probabilité de chute ou d'images indiquant la survenue de chute.

9. Système selon la revendication 1, dans lequel le dispositif de surveillance effectue une classification visant à déterminer si l'identité déterminée du patient appartient ou non soit à un groupe à haut risque de chute, soit à un groupe à haut risque de récupération d'anesthésie, et fait une prédiction avec une sensibilité accrue si le patient appartient au groupe à haut risque.

10. Procédé de prédiction de probabilité de chute ou de degré de récupération d'anesthésie, le procédé comprenant :
la capture d'une image par au moins une caméra installée à un emplacement préétabli dans un hôpital ;
la détection du mouvements d'un patient dans l'image ;
la reconnaissance du visage du patient dans l'image pour déterminer l'identité du patient, et la reconnaissance de l'expression et du regard du patient ; et
la prédiction de probabilité de chute ou de degré de récupération d'anesthésie en utilisant l'action du patient détectée dans la détection du mouvement du patient, l'identité du patient déterminée dans la reconnaissance de l'expression et du regard du patient, et l'expression et le regard du patient.
